(19) Europäisches Patentamt
European Patent Office
Office européen des brevets

(11) Veröffentlichungsnummer: **0 265 388**
**A1**

# (12) EUROPÄISCHE PATENTANMELDUNG

(21) Anmeldenummer: **87810604.6**

(22) Anmeldetag: **19.10.87**

(51) Int. Cl.$^4$: **C 07 D 207/325**
A 61 K 31/40

(30) Priorität: **24.10.86 CH 4235/86 19.01.87 CH 178/87**
**12.03.87 CH 925/87 12.03.87 CH 926/87**

(43) Veröffentlichungstag der Anmeldung:
**27.04.88 Patentblatt 88/17**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Ostermayer, Franz, Dr.**
**Am Hang 5**
**CH-4125 Riehen (CH)**

**Jaekel, Joachim, Dr.**
**Im Stigler 30**
**CH-4312 Magden (CH)**

**Veillon, Nicole**
**Steinbühlweg 21**
**CH-4123 Allschwil (CH)**

Die Bezeichnung der Erfindung wurde geändert (Richtlinien für die Prüfung im EPA, A-III, 7.3).
Patentansprüche für folgende Vertragsstaaten: ES + GR.

(54) **Pyrrol-Derivate, therapeutische Mittel, die sie enthalten und ihre Verwendung.**

(57) Die Erfindung betrifft (+)-1-Isopropylamino-3-[o-(pyrrol-1-yl)-phenoxy]-2-propanol und dessen Salze, seine Verwendung und Herstellung sowie diesen Wirkstoff enthaltende pharmazeutische Präparate und deren Herstellung. Ebenfalls Gegenstand der Erfindung ist die Verwendung des racemischen 1-Isopropylamino-3-[o-(pyrrol-1-yl)-phenoxy]-2-propanols oder eines pharmazeutisch verwendbaren, nichttoxischen Salzes davon und einen solchen Wirkstoff enthaltende pharmazeutische Präparate zur Behandlung von Angstzuständen.

EP 0 265 388 A1

**Beschreibung**

Therapeutische Mittel und ihre Verwendung

Die Erfindung betrifft (+)-1-Isopropylamino-3-[o-(pyrrol-1-yl)-phenoxy]-2-propanol und dessen Salze, seine Verwendung und Herstellung sowie diesen Wirkstoff enthaltende pharmazeutische Präparate und deren Herstellung. Ebenfalls Gegenstand der Erfindung ist die Verwendung des racemischen 1-Isopropylamino-3-[o-(pyrrol-1-yl)-phenoxy]-2-propanols oder eines pharmazeutisch verwendbaren, nichttoxischen Salzes davon und einen solchen Wirkstoff enthaltende pharmazeutische Präparate zur Behandlung von Angstzuständen.

In der Deutschen Offenlegungsschrift Nr. 2,409,313 wird unter anderem racemisches 1-Isopropylamino-3-[o-(pyrrol-1-yl)-phenoxy]-2-propanol beschrieben. Die in dieser Referenz offenbarten Substanzen können als Beta-Rezeptoren-Blocker, teilweise mit erregungshemmenden Eigenschaften, verwendet werden. Diese Eigenschaften wurden im Pargylin-Reserpin-Antagonismus-Test festgestellt. In diesem Testmodell wird die durch Testsubstanzen bewirkte Hemmung von unmotivierter Hyperaktivität bestimmt, die als Folge von pharmakogen-induzierter Noradrenalinausschüttung auftritt. Die Hemmung der so bewirkten Hyperaktivität tritt bei höheren Dosierungen, d.h. im gleichen Dosisbereich, in dem sich bei betarezeptorenblockierenden Eigenschaften zeigen. Als tägliche Wirkstoffdosierung wurden bei Verabreichung von Tabletten etwa 30 bis 240 mg vorgeschlagen.

Ueberraschend ist nun die Feststellung, dass (+)-1-Isopropylamino-3-[o-(pyrrol-1-yl)-phenoxy]-2-propanol in freier Form oder in Form eines pharmazeutisch verwendbaren, nichttoxischen Salzes keine relevante β-blockierende, jedoch ausgeprägte anxiolytische Eigenschaften aufweist. Zusätzlich weist das (+)-Enantiomere nootrope und antidepressive Aktivitäten auf.

Gegenstand der Erfindung ist also (+)-1-Isopropylamino-3-[o-(pyrrol-1-yl)-phenoxy]-2-propanol oder ein Salz, insbesondere ein pharmazeutisch verwendbares, nichttoxisches Salz davon.

Pharmazeutisch verwendbare Salze des jeweiligen Wirkstoffs sind dessen pharmazeutisch verwendbare Säureadditionssalze, beispielsweise mit starken anorganischen Säuren, wie Mineralsäuren, mit Sulfaminsäuren, wie Cycloniederalkylsulfaminsäuren, mit starken organischen Carbonsäuren, wie Niederalkancarbonsäuren, gegebenenfalls ungesättigten Dicarbonsäuren oder durch Hydroxy und/oder Oxo substituierten Niederalkancarbonsäuren, oder mit Sulfonsäuren, wie Niederalkan- oder gegebenenfalls substituierten Benzolsulfonsäuren, beispielsweise das entsprechende Sulfat, Phosphat, Hydrohalogenid, wie Hydrochlorid oder Hydrobromid, Cyclohexylsulfamat, Acetat, Malonat, Oxalat, Maleinat, Fumarat, Citrat, Tartrat, Pyruvat sowie Sulfonat, insbesondere Methansulfonat, ferner Benzol- oder p-Toluolsulfonat. Ein bevorzugtes pharmazeutisch verwendbares Salz des (+)-Enantiomeren ist das entsprechende Fumarat, während das Racemat bevorzugt als Hydrochlorid verwendet wird. Dabei sind unter mit "Nieder" bezeichneten Teilstrukturen, sofern nicht abweichend definiert, vorzugsweise solche zu verstehen, die bis und mit 7, vor allem bis und mit 4 Kohlenstoffatome (C-Atome) enthalten.

Infolge der engen Beziehung zwischem dem jeweiligen Wirkstoff in freier Form und in Form seiner pharmazeutisch verwendbaren Salze sind vorstehend und nachfolgend unter der freien Verbindung bzw. seinen pharmazeutisch verwendbaren Salzen sinn- und zweckgemäss gegebenenfalls auch die entsprechenden pharmazeutisch verwendbaren Salze bzw. die freie Verbindung zu verstehen.

Der jeweils erfindungsgemäss verwendbare Wirkstoff kann auch in Form seiner Hydrate vorliegen oder andere pharmazeutisch unbedenkliche Lösungsmittel, beispielsweise solche, die zur Kristallisation des entsprechenden Wirkstoffs, der in fester Form vorliegt, verwendet werden, einschliessen.

Das charakteristische anxiolytische Profil von (+)-1-Isopropylamino-3-[o-(pyrrol-1-yl)-phenoxy]-2-propanol ergibt sich beispielsweise aus den Ergebnissen des Geller-Testes, [J. Geller. Psychopharmacologia, 3, S. 374 ff. (1962)], im dem Ratten einer durch Fussschock induzierten Konfliktsituation ausgesetzt werden, erweist sich (+)-1-Isopropylamino-3-[o-(pyrrol-1-yl)-phenoxyl]-2-propanol als starkes Anxiolytikum. Bei diesem Test werden die Füsse der Verscuchstiere beim Bedienen eines Hebels zur Nahrungsfreigabe periodisch Elektroschocks ausgesetzt. Normalerweise wird nur bei Verabreichung von hohen Dosen an Anxiolytika die Reaktion auf die Bestrafung unterdrückt. Bei einer Gruppe von Ratten, die eine Dosis ab 3 mg/kg p.o. von (+)-1-Isopropylamino-3-[o-(pyrrol-1-yl)-phenoxy]-2-propanol erhalten haben, reagieren bereits ab etwa 80 % der Versuchstiere mit einem markanten Anstieg der Hebelbedienungshäufigkeit während der Schockphase.

Insbesondere konnten signifikante anxiolytische Effekte bei Verabreichung des Wirkstoffs in sozialen Konfliktsituationen bei Rhesusaffen festgestellt werden. Die experimentalle Grundlage für diesen Sozialkonflikt-Test analog J. Jaekel in Biologische Psychiatrie (Forschungsergebnisse), W. Kemp (Hrsg.), Springer Verlag, Heidelberg, 1986, ist die hierarchische Rangordnung bei Rhesusaffen. Der relative Rangstatus eines Tieres ist der bestimmende Faktor für interindividuelle Kommunikation, z.B. für Aggression oder wechselseitiges soziales Putzen. Normalerweise werden Tiere mit geringem Rangstatus von höher gestellten Grupenmitgliedern unterdrückt. Diese unterdrückten Tiere ziehen sich in eine soziale Isolierung zurück und vermeiden ängstlich jeden Sozialkontakt. Wiederholte Wirkstoffgaben in Dosen von 0,2 mg/kg p.o. bei Gruppen von 5 bzw. 7 Affen führten zu einer signifikanten Reduktion des ängstli chen Verhaltensmuster der jeweiligen Affen, die in der Rangordnung am niedrigsten eingeordnet sind. Ebenso nahmen sie aktiv soziale Kontakte auf und wurden zu Partnern beim Pflegen, obwohl sie vorher niemals Pflegepartner waren. Diese

Effekte werden als deutliche Anxiolyse angesehen.

In Testmodellen an Rhesusaffen sowie an Ratten konnten weiterhin eine zusätzliche nootrope und antidepressive Wirkungskomponente festgestellt werden. So bewirkte (+)-1-Isopropylamino-3-[o-(pyrrol-1-yl)-phenoxy]-2-propanol in einer Doppelblindstudie bei Rhesusaffen gegen Placebo im "Delayed-Matching-to-Sample-Test" (Zeit-verzögerte-Wahl-nach-Muster) gemäss der Methodik von H. F. Harlow et al., J. Comp. Physiol. Psychol. 53, 113 - 121 (1960) z.B. mit 1 mg/kg p.o. eine signifikant erhöhte Erfolgsrate im Defizitbereich. Dieser bedeutet diejenige Zeitverzögerung in Sekunden, nach der ein Affe einen Diskriminationserfolg von < 75 % hat. Weiterhin konnten die Vorzögerungszeiten für den Defizitbereich um durchschnittlich 20 Sekunden erhöht werden. Neben diesen nootropen Effekten konnten in diesem Test auch antidepressive Eigenschaften beobachtet werden. Die Rhesusaffen liessen nämlich deutliche Motivations- und Antriebssteigerungen erkennen, da ihr Interesse an den gestellten Aufgaben und ihre Bereitschaft mitzuarbeiten deutlich zunahm.

Die nootrope bzw. antidepressive Wirkungskomponente konnten ebenfalls bei Untersuchungen mit Ratten manifestiert werden. So wurden in einem komplizierten Tieflabyrinth analog dem Modell von J.B. Watson, Animal Education, Contrib. Psychol. Lab. Univ. Chicago, 4, 5 - 122 (1903) infolge geringerer Fehlerquote eine deutliche Verbesserung der Orientierungsleistungen der Ratten festgestellt (nootrope Wirkung). Auf motivations- bzw. antriebssteigernde Effekte weisen die im Vergleich mit Placebotieren signifikant verkürzten Laufzeiten der Ratten vom Start zum belohnten Ziel, was auf antidepressive Eingenschaften der Wirksubstanz schliessen lässt.

Ferner bewirkt (+)-1-Isopropylamino-3-[o-(pyrrol-1-yl)-phenoxy]-2-propanol an Ratten im Screening-Modell nach S. Bischoff et al., Europ. J. of Pharmacology 104, 173 - 176 (1984), in vivo dosisabhängig Erhöhungen von $^{3}H$-Spiperon-Bindung stratialer Dopamin-Rezeptoren.

Somit weist (+)-1-Isopropylamino-3-[o-(pyrrol-1-yl)-phenoxy]-2-propanol ein ausgeprägtes und spezifisches anxiolytisches Profil auf, da es beispielsweise aggressives Verhalten reduziert und vor allem zu einer Verbesserung des positiven Sozialverhaltens führt. Weiterhin wirkt dieses (+)-Enantiomere deutlich nootrop und antidepressiv. Dementsprechend kann (+)-1-Isopropylamino-3-[o-(pyrrol-1-yl)-phenoxy]-2-propanol oder ein pharmazeutisch verwendbares, nichttoxisches Salz davon bei der Behandlung von Angstzuständen, insbesondere von generalisierten (chronischen) Angstzuständen, panic disorders, obsessive compulsive behavior and Phobien, wie sozialen Phobien, Neophobie (Angst vor Neuem) und Agoraphobie (Platzangst), bei der Behandlung von Dementien jeglicher Art, z.B. der Alzheimer Krankheit und von altersabhängigen und traumatisch bedingten Defiziten des Lernens und des Gedächtnisses, sowie bei der Behandlung von Depressionen, verwendet werden.

Weiterer Gegenstand der Erfindung ist somit die Verwendung von (+)-1-Isopropylamino-3-[o-(pyrrol-1-yl)-phenoxy]-2-propanol oder eines pharmazeutisch verwendbaren, nichttoxischen Salzes zur Behandlung von Angstzuständen, von Dementien jeglicher Art und von Depressionen und zur Herstellung von pharmazeutischen Präparaten zur Behandlung von Angstzuständen, von Dementien jeglicher Art und von Depressionen.

Ein weiterer Gegenstand der Erfindung sind Verfahren zur Herstellung von (+)-1-Isopropylamino-3-[o-(pyrrol-1-yl)-phenoxy]-2-propanol und seiner Salze, welche in an sich bekannter Weise durchgeführt werden können und z.B. dadurch gekennzeichnet sind, dass man

a) racemisches 1-Isopropylamino-3-[o-(pyrrol-1-yl)-phenoxy]-2-propanol oder ein Salz davon in das (+)-Enantiomere auftrennt, oder

b) ein Enantiomeres der Formel

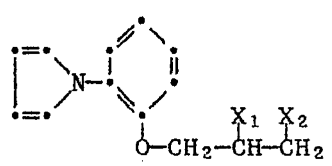

(I),

worin einerseits $X_1$ und $X_2$ gemeinsam für -O- stehen oder andererseits $X_1$ Hydroxy und $X_2$ reaktionsfähiges veresteres Hydroxy, vor allem Halogen oder Sulfonyloxy, bedeutet, mit Isopropylamin umsetzt, oder

c) das (R)-Enantiomere der Formel

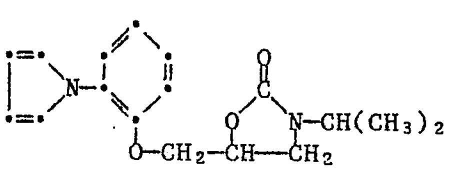

(II)

hydrolisiert, oder

d) von der (R)-enantiomeren Verbindung der Formel

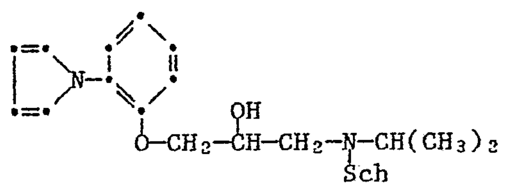

(III),

worin Sch für eine üblicherweise zum Schutz von Aminogruppen verwendete Gruppe bedeutet, die Schutzgruppe abspaltet, oder

e) ausgehend von dem (R)-Enantiomeren der Formel

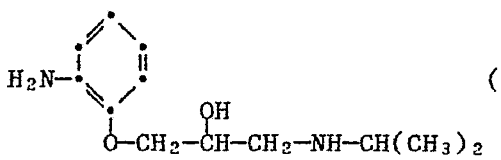

(IV),

den Pyrrol-1-yl-Ring aufbaut, und/oder, erhältliches freies (+)-1-Isopropylamino-3-[o-(pyrrol-1-yl)-phenoxy]-2-propanol in ein Salz überführt oder ein erhältliches Salz von (+)-1-Isopropylamino-3-[o-(pyrrol-1-yl)-phenoxy]-2-propanol in das freie Enantiomere überführt.

Die vor- und nachstehend in den Varianten beschriebenen Umsetzungen werden in an sich bekannter Weise durchgeführt, z.B. in Ab- oder üblicher Weise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -10° bis zur Siedetemperatur des Reaktionsmediums, vorzugsweise von etwa -10° bis etwa 150°C, und, falls erforderlich, in einem geschlossenen Gefäss, unter Druck, in einer Inertgasatmosphäre und/oder unter wasserfreien Bedingungen arbeitet.

Das vor- und nachstehend aufgeführte Ausgangsmaterial der Formeln I, II und III, das für die Herstellung der erfindungsgemässen Verbindung und ihrer Salze entwickelt wurde, ist zum Teil bekannt oder kann ebenfalls nach an sich bekannten Methoden, z.B. analog den vorstehend beschriebenen Verfahrensvarianten, hergestellt werden.

Ausgangsmaterialien mit basischem Zentrum können z.B. in Form von Säureadditionssalzen, beispielsweise mit den vorstehend im Zusammenhang mit Salzen der erfindungsgemässen Verbindung aufgeführten Säuren, vorliegen.

Im Rahmen der vor- und nachstehenden Verfahrensbeschreibung bedeutet reaktionsfähiges verestertes Hydroxy, sofern nicht abweichend definiert, insbesondere mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes $C_1$-$C_7$-Alkansulfonyloxy, z.B. Methan- oder Trifluormethansulfonyloxy, $C_3$-$C_7$-Cycloalkan sulfonyloxy, z.B. Cyclohexansulfonyloxy, oder gegebenenfalls, z.B. durch $C_1$-$C_7$-Alkyl oder Halogen, substituiertes Benzolsulfonyloxy, z.B. p-Bromphenyl-oder p-Toluolsulfonyloxy.

Als Aminoschutzgruppe Sch kommt insbesondere Arylmethyl, wie Mono-, Di- oder Triphenylmethyl, in erster Linie Benzyl, in Frage.

Werden bei den vor- und nachstehend beschriebenen Umsetzungen beispielsweise Basen verwendet, so kommen, sofern nicht abweichend aufgeführt, beispielsweise Alkalimetallhydroxide, -hydride, -amide, -alkanolate, -carbonate, -triphenylmethylide, -di-$C_1$-$C_7$-alkylamide, -amino-$C_1$-$C_7$-alkylamide oder -$C_1$-$C_7$-alkylsilylamide, Naphthalinamine, $C_1$-$C_7$-Alkylamine, basische Heterocyclen, Ammoniumhydroxide sowie carbocyclische Amine in Frage. Beispielhaft seien Lithiumhydroxid, Natrium-hydroxid, -hydrid, -amid, -ethylat, Kalium-tertbutylat, -carbonat, Lithiumtriphenylmethylid, -diisopropylamid, Kalium-3-(aminopropyl)-amid, -bis-(trimethylsilyl)-amid, Dimethylaminonaphthalin, Di- oder Triethylamin, Pyridin, Benzyl-trimethylammoniumhydroxid, 1,5-Diaza-bicyclo[4.3.0]non-5-en (DBN) sowie 1,8-Diazo-bicyclo[5.4.0]undec-7-en (DBU) genannt.

Die vorstehenden Verfahrensvarianten können je nach Wahl des Ausgangsmaterials so geführt werden, dass die jeweilige Synthese enantioselektiv verläuft, d.h. eines der möglichen Enantiomeren ausschliesslich bzw. überwiegend erhalten werden kann.

Variante a):

Racemisches 1-Isopropylamino-3-[o-pyrrol-1-yl)phenoxy]-2-propanol lässt sich nach bekannten Methoden in die optischen Antipoden zerlegen, beispielsweise durch Umkristallisation aus einem optisch aktiven Lösungsmittel, Chromatographie an chiralen Absorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenether, wobei nur ein Enantiomeres komplexiert wird, oder durch Ueberführung in diastereomere Salze, z.B. durch Umsetzung des basischen Racemats mit einer optisch aktiven Säure, wie

Carbonsäure, z.B. Mandel-, Wein-oder Aepfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann.

Variante b):

Stehen $X_1$ und $X_2$ der Formel I für -O-, wird das entsprechende (R)-Enantiomere verwendet. Die Umsetzung dieses Enantiomeren wird bevorzugt mit einem Ueberschuss an Isopropylamin durchgeführt.

Für die Umsetzung der Verbindung der Formel I , worin $X_2$ vorzugsweise Halogen, wie Chlor oder Brom, ferner Sulfonyloxy, bedeutet und $X_1$ Hydroxy ist, wird die (S)-enantiomere Form eingesetzt. Die Ueberführung eines derartigen Enantiomeren der Formel I in das erfindungsgemässe Enantiomere kann beispielsweise in Gegenwart einer der vorstehend genannten anorganischen Basen erfolgen, in erster Linie wird jedoch mit einem Ueberschuss an Isopropylamin gearbeitet.

Variante c):

Die Hydrolyse des (R)-Enantiomeren der Formel II kann insbesondere in Gegenwart einer Base erfolgen, in erster Linie durch alkalische Hydrolyse, z.B. in Gegenwart eines Alkalimetallhydroxids, wie Natronlauge.

Variante d):

Die Abspaltung der Aminoschutzgruppe Sch aus der (R)-enantiomeren Verbindung der Formel III erfolgt in an sich bekannter Weise erfolgen. So kann z.B. Benzyl Sch durch katalytische Hydrierung abgespalten werden.

Als Hydrierungskatalysatoren kommen z.B. Elemente der VIII. Nebengruppe des Periodensystems der Elemente oder deren Derivate in Betracht, wie Palladium, Platin, Platinoxid, Ruthenium, Rhodium, Tris(triphenylphosphin)-rhodium-I-halogenid, z.B. -chlorid, oder Raney-Nickel, die gegebenenfalls auf einem Trägermaterial, wie Aktivkohle, Alkalimetallcarbonat bzw. -sulfat oder einem Kieselgel, aufgezogen sind. Als bevorzugter Katalysator wird Palladium verwendet.

Verfahren e):

Der Aufbau des Pyrrolrings ausgehend von der Verbindung der Formel IV erfolgt in an sich bekannter Weise. So kann man beispielsweise die Verbindung der Formel IV mit einem 2,5-Diniederalkoxytetrahydrofuran, z.B. 2,5-Dimethoxy-tetrahydrofuran, vorteilhaft in Gegenwart einer Säure, z.B. Essigsäure, und unter Erwärmen, z.B. auf Rückflusstemperatur des Reaktionsmediums, umsetzen.

Salze der erfindungsgemässen Verbindung können in an sich bekannter Weise hergestellt werden. So erhält man beispielsweise entsprechenden Säureadditionssalze durch Behandeln mit einer Säure oder einem geeigneten Ionenaustauscherreagenz. Salze können in üblicher Weise in die freien Verbindungen überführt werden, Säureadditionssalze z.B. durch Behandeln mit einem geeigneten basischen Mittel.

Je nach Verfahrensweise bzw. Reaktionsbedigungen können die erfindungsgemässen Verbindung mit salzbildenden, insbesondere basischen Eigenschaften, in freier Form oder bevorzugt in Form von Salzen erhalten werden.

Das in den Verfahrensvarianten aufgeführte Ausgangsmaterial ist zum Teil bekannt bzw. in an sich bekannter Weise herstellbar.

Die Herstellung von racemischem 1-Isopropylamino-3-[o-(pyrrol-1-yl)-phenoxy]-2-propanol wird z.B. in DE-OS Nr. 2,409,313 beschrieben.

Die Verbindung der Formel I, worin $X_1$ und $X_2$ für -O- stehen, kann beispielsweise hergestellt werden, in dem man z.B. von (R)-2,2-Dimethyl-1,3-dioxolan-4-methanol-p-toluolsulfonat ausgeht und mit einem Alkalimetallsalz von 2-(Pyrrol-1-yl)-phenol zu (S)-2,2-Dimethyl-4-{[o-(pyrrol-1-yl)-phenoxy]-methyl}-1,3-dioxolan umsetz.

Nach saurer Hydrolyse, Bildung des Tosylats und anschliessender Behandlung mit einer der vorstehend genannten Basen, z.B. Natriumethylat, ist das (R)-1,2-Epoxy-3-[o-(pyrrol-1-yl)-phenoxy]-propan der Formel I zugänglich. Das nach saurer Hydrolyse des (S)-2,2-Dimethyl-4-{[o-(pyrrol-1-yl)-phenoxy]-methyl}-1,3-dioxolan erhältliche Diol, d.h. eine Verbindung der Formel I, worin $X_1$ und $X_2$ Hydroxy bedeuten, kann vorteilhafterweise auch durch Umsetzung mit einem Orthoester, z.B. dem Orthoessigsäuretrimethylester, in die entsprechende cyclische Orthoesterform übergeführt werden, dessen Ringöffnung, z.B. Trimethylchlorsilan, und anschliessende Cyclisierung des so erhältlichen Chlorhydrins mit einer Base, z.B. Natronlauge, zur entsprechenden Verbindung der Formel I führt, worin $X_1$ und $X_2$ gemeinsam -O- bilden.

Die Verbindung der Formel II ist z.B. herstellbar, indem man (R)-3-Isopropylamino-4-(p-toluolsulfonyloxymethyl)-oxazolidin-2-on mit o-(Pyrrol-1-yl)-phenol in Anwesenheit einer Base, z.B. Natriumhydrid, und eines Lösungsmittels, z.B. DMF, umsetzt.

Zur Herstellung einer Verbindung der Formel III kann man beispielsweise von (R)-1,2-Epoxy-3-[o-(pyrrol-1-yl)-phenoxy]-propan ausgehen und dieses mit entsprechend geschütztem Isopropylamin, z.B. N-Benzylisopropylamin, umsetzen.

Zur Herstellung der Verbindung der Formel IV kann man beispielsweise von 2-Nitrophenol ausgehen, dieses zunächst mit (S)-Glycidol kondensieren und anschliessend mit Isopropylamin behandeln. Im nächsten Reaktionsschritt kann die Nitrogruppe, beispielsweise durch katalytische Hydrierung in Gegenwart eines Hydrierungskatalysators, z.B. Pd/C oder Raney-Nickel, zur Aminogruppe reduziert werden.

Bei der Herstellung der jeweiligen Ausgangsverbindungen kann infolge ines Substituentenwechsels verbunden mit einem Prioritätswechsel die Bezeichnung der Konfiguration wechseln, auch wenn keine stereochemischen Veränderungen direkt am Chiralitätzentrum stattfinden. Die enantioselektive Synthese wird jedoch jeweils so geführt, dass diejenigen enantiomeren Ausgangsmaterialien hergestellt werden, die direkt zu dem erfindungsgemässen Enantiomeren führen. Ferner kann racemisches Ausgangsmaterial in die entsprechende erforderliche Form durch übliche Racematspaltung aufgetrennt werden.

Weiterhin wurde überraschend gefunden, dass das Racemat 1-Isopropylamino-3-[o-(pyrrol-1-yl)-phenoxy]-2-propanol und entsprechende pharmazeutisch verwendbare, nichttoxische Salze davon neben den mit der β-Blockade zusammenhängenden Wirkungen als zusätzliche Wirkungskomponente ausgeprägte anxiolytische Aktivitäten aufweist. Diese anxiolytischen Eigenschaften treten bereits bei Dosierungen auf, bei denen noch keine Beta-Rezeptoren-hemmenden Effekte auftreten. 1-Isopropylamino-3-[o-(pyrrol-1-yl)-phenoxy]-2-propanol besitzt somit die zusätzliche Eigenschaft, durch Angst induzierte Hyperaktivität zu normalisieren bzw. in Angstzuständen auftretendes gehemmtes Verhalten in eine Antriebsteigerung umzuwandeln, und besitzt dementsprechend auch eine "Antianst-Wirkung".

Das charakterische anxiolytische Profil von 1-Isopropylamino-3-[o-(pyrrol-1-yl)-phenoxy]-2-propanol ergibt sich beispielsweise aus der Hemmung der Hyperaktivität von Ratten, die in Isolation gehalten werden, gemessen an ihrem lokomotorischen und exploratorischen Verhalten, wobei maximale Effekte bei einer Dosierung von 0,01-0,1 mg/kg p.o. erzielt werden [analog der Methodik von R. Weinstock et al., Psychopharmacologia 30, 241ff. (1973). Die Hyperaktivität von isoliert gehaltenen Ratten konnte dagegen im Falle des Propranolols erste bei einer Dosis von 0,3 mg/kg p.o. maximal gehemmt werden.

Auch in dem vorstehend beschriebenen Geller-Test erweist sich 1-Isopropylamino-3-[o-(pyrrol-1-yl)-phenoxy]-2-propanol als starkes Anxiolytikum. Bei einer Gruppe von 29 Ratten, die eine Dosis von 20 mg/kg p.o. von 1-Isopropylamino-3-[o-(pyrrol-1-yl)-phenoxy]-2-propanol erhalten haben, reagieren bereits 50 % der Versuchstiere mit einem markanten Anstieg der Hebelbedienungshäufigkeit während der Schockphase, wobei der Anstieg bei einzelnen Tieren 300 % betrug. In diesem Fussschocktest führte eine Dosierung von 20 mg/kg Propanolol p.o. bei Ratten zu keiner signifikanten Verhaltensänderung.

Insbesondere konnten signifikante anxiolytische Effekte bei Verabreichung des Wirkstoffs in sozialen Konfliktsituationen bei Rhesusaffen im oben beschriebenen Sozialkonflikt-Test nach Jaekel festgestellt werden. Wiederholte Wirkstoffgaben in Dosen von 0,1-0,3 mg/kg p.o. bie Gruppen von 7 Affen führte zu einer signifikanten Reduktion des ängstlichen Verhaltensmuster der Affen, die in der Rangordnung am niedrigsten eingeordnet sind, und zu einer Zunahme der Sozialkontakte. Ebenso nahmen sie aktiv Kontakt auf und wurden zu Partnern beim Pflegen, obwohl sie vorher niemals Pflegepartner waren. Diese Effekte wurden sowohl bei Gruppen mit hohem Niveau an Sozialkontakten als auch bei Gruppen mit wenig Pflegepartnerschaften beobachtet. Nach Verabreichung von 0,3-3,0 mg/kg p.o. Oxprenolol konnte im Verhaltensmuster der Rhesusaffen keine Steigerung der Sozialkontakte von Affen mit geringer Rangordnung beobachtet werden. Bei einer Dosierung 0,5-10,0 mg/kg p.o. Propranolol wurden die Sozialkontakte der Rhesusaffen mit niedrigem Rangstatus relativ wenig beeinflusst. Wie zu erwarten wurde dieses ausgeprägte anxiolytische Profil bei den Standard-Beta-Rezeptoren-Blockern Oxprenolol nicht und bei Propanolol in weitaus geringerem Masse erst bei Beta-Rezeptoren blockierenden Dosierungen festgestellt. Dagegen weist das Racemat diesen Effekt in nicht β-blockierend wirkenden Dosierungen auf.

Der erfindungsgemäss zu verwendende Wirkstoff ist weiterhin ein potenter selektiver Antagonist des präsynaptischen Serotonin-1B-Rezeptors ($5\text{-}HT_{1B}$), während Propranolol und Oxprenolol weniger selektiv wirken, da diese ebenso mit den postsynaptischen $5\text{-}HT_{1A}$-Rezeptor reagieren.

Die vorstehend aufgeführten pharmakologischen Untersuchungen dokumentieren also, dass 1-Isopropylamino-3-[o-(pyrrol-1-yl)-phenoxy]-2-propanol eine signifikante anxiolytische Aktivität aufweist, und diese sich auch bei niedrigen Dosierungen manifestiert und somit eine deutliche Differenzierung zwischen anxiolytischer und Beta-Rezeptoren-hemmender Aktivität auftritt.

Ueberraschend ist also die Tatsache, dass das anxiolytische Profil dieses Wirkstoffs auch bei nicht-betarezeptorenblockierend wirkenden Dosierungen sowohl im Tiermodell als auch am Menschen überaus ausgeprägt ist. Letzteres bestätigen Untersuchungen an stationären Patienten.

Aufgrund einer Dreizentren-Doppelblindstudie mit mehr als 100 stationären Patienten kann als überraschend angesehen werden, dass bei Verabreichung einer Tagesdosis von 2 mg p.o. signifikante, mindestens ebenso potente anxiolytische Effekte erzielt wurden wie bei Verabreichung einer Tagesdosis von 4 mg p.o. des erfindungsgemäss zu verwendenden Wirkstoffs.

Ferner wurde festgestellt, dass beispielsweise bei niedrigen Tagesdosen, wie 2 mg und 4 mg p.o., keine cardiovaskulären Effekte aufgetreten sind, d.h. keine β-Blockade festgestellt wurde. Entsprechendes ist bei der Verwendung des (+)-Enantiomeren zu erwarten.

Die Behandlung von Angstzuständen beim Menschen kann dementsprechend bevorzugt bei nicht-betarezeptorenblockierenden Dosierungen erfolgen. Dies bedeutet einerseits eine höhere Selektivität bei der Therapie, andererseits ist dadurch auch eine beträchtliche Reduktion von Nebenwirkungen, die durch hohe Wirkstoffdosen hervorgerufen werden können, zu erwarten.

Dementsprechend kann 1-Isopropylamino-3-[o-(pyrrol-1-yl)-phenoxy]-2-propanol oder ein pharmazeutisch verwendbares, nichttoxisches Salz davon bei der Behandlung von Angstzuständen, insbesondere von generalisierten (chronischen) Angstzuständen, panic disorders, obsessive compulsive behavior und Phobien, wie sozialen Phobien, Neophobie (Angst vor Neuem) und Agoraphobie (Platzangst), verwendet werden.

In den vorstehend angeführten Testanordnungen nach H. F. Harlow et al. und J. B. Watson konnten bei den verwendeten Dosierungen keine aussagefähigen Beobachtungen hinsichtlich nootroper und antidepressiven Eigenschaften von racemischem 1-Isopropylamino-3-[o-(pyrrol-1-yl)-phenoxy]-2-propanol gemacht werden.

Weiterer Gegenstand der Erfindung ist somit die Verwendung von 1-Isopropylamino-3-[o-(pyrrol-1-yl)-phenoxy]-2-propanol oder eines pharmazeutisch verwendbaren, nichttoxischen Salzes zur Behandlung von Angstzuständen und zur Herstellung von pharmazeutischen Präparaten zur Behandlung von Angstzuständen.

Die vorliegende Erfindung betrifft ebenso pharmazeutische Präparate, die als Wirkstoff (+)-1-Isopropylamino-3-[o-(pyrrol-1-yl)-phenoxy]-2-propanol, der in reiner bzw. im wesentlichen reiner Form vorliegt, oder ein pharmazeutisch verwendbares, nichttoxisches Salz davon enthalten, ebenso für die Behandlung von Angstzuständen bestimmte pharmazeutische Präparate die racemisches 1-Isopropylamino-3-[o-(pyrrol-1-yl)-phenoxy]-2-propanol oder ein pharmazeutisch verwendbares, nichttoxisches Salz davon enthalten, sowie Verfahren zur Herstellung solcher pharmazeutischer Präparate.

Bei den erfindungsgemässen pharmazeutischen Präparaten, welche die erfindungsgemäss zur Verwendung vorgeschlagene Verbindung oder ein pharmazeutisch verwendbares Salz davon enthalten, handelt es sich um solche zur enteralen, wie oralen, ferner rektalen, und parenteralen Verabreichung an Warmblüter(n), wobei der pharmakologische Wirkstoff allein oder zusammen mit einem pharmazeutisch anwendbaren Trägermaterial enthalten ist.

Die neuen pharmazeutischen Präparate enthalten z.B. einen therapeutischen z.B. anxiolytischen bzw. nootropen sowie antidepressiven Effekt erzeugende Menge, wie ab etwa 0,5 %, vorzugsweise ab 1,0 %, des Wirkstoffs. Erfindungsgemässe pharmazeutische Präparate zur enteralen bzw. parenteralen Verabreichung sind z.B. solche in Dosis einheitsformen, wie Dragées, Tabletten, Kapseln oer Suppositorien, ferner Ampullen. Diese werden in an sich bekannter Weise, z.B. mittels konventioneller Misch-, Granulier, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferne Bindemittel, wie Stärkekleister, unter Verwendung von Mais-, Weizen-, Reis- oder Kartoffelstärke, Gelatine, Tragakanth, Methylcellulose und/oder Polyvinylpyrrolidon, wenn erwünscht, Sprengmittel, wie die obengenannten Stärken, ferner Carboxmethylstärke, quervernetzes Polyvinylpyrrolidon, Agar, Alginsäure oder ein Salz davon, wie Natriumalginat. Hilfsmittel sind in erster Linie Fliess-, Regulier- und Schmiermittel, z.B. Kieselsäure, Talk, Stearinsäure oder Salze davon, wie Magnesium- oder Calciumstearat, und/oder Polyethylenglykol, Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kanseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositorienmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat oder Triglyceride, verwendet oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Gegenstand der Erfindung ist weiterhin ein Verfahren zur Herstellung von entsprechenden pharmazeutischen Präparaten, z.B. zur Behandlung von Angstzuständen, welches dadurch gekennzeichnet ist, dass man, in an sich bekannter Weise, (+)-1-Isopropylamino-3-[o-(pyrrol-1-yl)-phenoxy]-2-propanol bzw. das entsprechende Racemat oder ein pharma zeutisch verwendbares, nichttoxisches Salz davon gegebenenfalls unter Beimischung von üblichen Hilfs- und Trägerstoffen zu entsprechenden Präparaten verarbeitet.

Die Dosierung des jeweiligen Wirkstoffs hängt von der Warmblüter-Spezies, dem Alter und dem

individuellen Zustand sowie der Applikationsweise ab. Entsprechende pharmazeutische Präparate werden bevorzugt einmal pro Tag verabreicht.

So können beim Menschen beispielsweise Tagesdosen von kleiner gleich 40 mg p.o. eingesetzt werden. Insbesondere werden Tagesdosen von etwa 10 bis 0,5 mg, beispielsweise etwa 10 bis 4 mg, in erster Linie jedoch 3,5 bis 0,5 mg, besonders bevorzugt 2 mg, p.o. verabreicht. Um auf Beta-Rezeptoren-blockierende Effekte zurückführende kardiovaskuläre Erscheinungen zu vermeiden, werden Tagesdosen von 4 bzw. 3,5 bis 0,5 mg, in erster Linie 2 mg, p.o. empfohlen.

Die nachfolgenden Beispiele illustrieren die oben beschriebene Erfindung und sollen die Bereitstellung einer typischen Applikationsform erläutern, jedoch keinesfalls die einzigen Ausführungs-und Applikationsformen darstellen. Ausserdem soll die Erfindung durch die Beispiele in ihrem Umfang in keiner Weise eingeschränkt werden.

Temperaturen werden in Celsiusgraden angegeben.

Herstellungsbeisipel 1:

27,4 g (100 mMol) racemisches 1-Isopropylamino-3-[o-(pyrrol-1-yl)-phenoxy]-2-propanol und 16,7 g (110 mMol) L-(+)-Mandelsäure werden in 143 ml Wasser gelöst und über Nacht auskristallisieren lassen. Die Kristalle werden abgesaugt und mehrmals aus möglichst wenig Wasser umkristallisiert, bis der Smp. 122-123° beträgt und ein Muster der freigesetzten Base eine optische Drehung von $[\alpha]^{20}_{D}$ : + 12±1° (c = 5 % in Methanol) aufweist. Man erhält so (+)-1-Isopropylamino-3-[o-(pyrrol-1-yl)-phenoxy]-2-propanol als Salz der (+)-Mandelsäure, dessen Enantiomeren-Reinheit mittels $^{13}$C-NMR als ≧ 99 % bestimmt wurde (90 MHz, $CDCl_3$).

Die Base wird hierauf mit wässriger Natronlauge freigesetzt und mit Ethylacetat extrahiert.

Herstellungsbeispiel 2: Herstellung des neutralen Fumarates

6,4 g (23 mM) rohes (+)-1-Isopropylamino-3-[o-(pyrrol-1-yl)-phenoxy]-2-propanol und 1,45 g (12,5 mM) Fumarsäure werden in 45 ml Isopropanol durch Erwärmen gelöst. Nach dem Abkühlen kristallisiert neutrales (+)-1-Isopropylamino-3-[o-(pyrrol-1-yl)-phenoxy]-2-propanol-fumarat vom Spm. 165-166°, $[\alpha]^{20}_{D}$ : + 19,4±0,2° (c = 5,4 % in Wasser).

Herstellungsbeispiel 3:

Man kann für die Enantiomeren-Trennung vorteilhafterweise auch von einem Produkt ausgehen, bei dem das eine Enantiomere, z.B. das (+)-Enantiomere bereits angereichert ist.

So kristallisiert z.B. aus dem racemischen 1-Isopropylamino-3-[o-(pyrrol-1-yl)-phenoxy]-2-propanol mit (-)-Mandelsäure vorzugsweise das (-)-Enantiomere. Durch alkalisch stellen mittels Natronlauge kann aus der Mutterlauge die an (+)-Enantiomeren angereicherte Base isoliert werden und mit (+)-Mandelsäure in das reine (+)-Enantiomere übergeführt werden (analog Beispiel 1).

Herstellungsbeispiel 4:

Eine Lösung von 9,8 g (0,045 Mol) (R)-1,2-Epoxy-3-[o-(pyrrol-1-yl)-phenoxy]-propan und 26,9 g (0,455 Mol) Isopropylamin in 100 ml Isopropanol wird 15 Stunden bei Raumtemperatur stehen gelassen. Durch Abdampfen der flüchtigen Anteile erhälte man rohes (R)-1-Isopropylamino-3-[o-(pyrrol-1-yl)-phenoxy]-2-propanol als gelbliches Oel, das eine Drehung $[\alpha]^{20}_{D}$ : + 11,8° ± 0,2° aufweist [c = 5 % in Methanol]. Es bildet ein neutrales Fumarat vom Smp. 165° (aus Isopropanol), $[\alpha]^{20}_{D}$ : + 19,0° ± 0,2° (c = 5 % in Wasser).

Das Ausgangs-Epoxid kann nach in der Literatur beschriebenen Verfahren hergestellt werden:

a) Aus o-(Pyrrol-1-yl)-phenol und (R)-2,2-Dimethyl-1,3-dioxolan-4-methanol-p-toluolsulfonat erhält man mittels Natriumhydrid in DMF das (S)-2,2-Dimethyl-4-{[o-(pyrrol-1-yl)-phenoxy]-methyl}-1,3-dioxolan vom Smp. 57 - 58° (aus Petroläther, $[\alpha]^{20}_{D}$ : + 24,0° ± 0,2° (c = 5 % in Methanol).

b) Hydrolyse der unter a) beschriebenen Verbindung in 80%iger Essigsäure während 30 Minuten bei 80° ergeben das (R)-3-[o-(Pyrrol-1-yl)-phenoxy]-propan-1,2-diol vom Smp. 95 - 96° (aus Aether), $[\alpha]^{20}_{D}$ : + 1,6° (c = 5,5 % in Methanol).

c) Das unter b) beschriebene Diol wird mit Orthoessigsäure-trimethylester unter Trifluoressigsäure-Katalyse zum (2 R/S, 4S)-2-Methoxy-2-methyl-4-{[o-(pyrrol-1-yl)phenoxy]-methyl}-1,3-dioxolan umgesetzt, welches roh weiter verwendet wird.

d) Der unter c) beschriebene, rohe cyclische Orthoester wird nach M.S. Newman et al., J. Org. Chem. 1978 38, 4203 mit Trimethylchlorsilan in das Chlorhydrin-Derivat übergeführt, welches durch Umsetzen mit 2-n. Natronlauge das (R)-1,2-Epoxy-3-[o-(pyrrol-1-yl)-phenoxy]-propan ergibt. Das Rohprodukt wird durch "flach"-Chromatographie an Kieselgel (Elution mit Toluol) gereinigt. Das so erhaltene farblose Oel zeigt eine opt. Drehung von $[\alpha]^{20}_{D}$ :-25,8° ± 0,2°; $[\alpha]^{20}_{365}$ :-81,6° (c = 5,3 % in Methanol).

Formulierungsbeispiel 1:

Filmdragées enthaltend als Wirkstoff neutrales (+)-1-Isopropylamino-3-[o-(pyrrol-1-yl)-phenoxy]-2-propanol-fumarat oder racemisches 1-Isopropylamino-3-[o-(pyrrol-1-yl)-phenoxy]-2-propanolhydrochlorid

Zusammensetzung:

Kern:

| | |
|---|---|
| Wirkstoff | 0,5 mg |
| Silica-Aerogel (Aerosil 200) | 2,5 mg |
| Cellulose (Avicel PH 102) | 36,0 mg |
| Laktose, krist. | 180,0 mg |
| Magnesiumstärke | 1,5 mg |
| Natriumcarboxymethylstärke | 19,5 mg |
| | 240,0 mg |

Beschichtung:

| | |
|---|---|
| Hydroxypropylmethylcellulose (Cellulose-HP-M-603) | 3,30 mg |
| Glycerylpolyethylenglycoloxystearat (Cremophor RH 40) | 0,15 mg |
| Eisenoxid, red. | 0,19 mg |
| Talkum (PH) | 2,93 mg |
| Titandioxid (PH) | 0,43 mg |
| | 7 mg |
| Gesamtgewicht | 247 mg |

Formulierungsbeispiel 2:

Filmdragées enthaltend als Wirkstoff neutrales (+)-1-Isopropylamino-3-[o-(pyrrol-1-yl)-phenoxy]-2-propanol-fumarat oder racemisches 1-Isopropylamino-3-[o-(pyrrol-1-yl)-phenoxy]-2-propanol-hydrochlorid

Zusammensetzung:

Kern:

| | |
|---|---|
| Wirkstoff | 2,0 mg |
| Silica-Aerogel (Aerosil 200) | 2,5 mg |
| Cellulose (Avicel PH 102) | 36,0 mg |
| Laktose, krist. | 180,0 mg |
| Magnesiumstearat | 1,5 mg |
| Natriumcarboxymethylstärke | 18,0 mg |
| | 240,0 mg |

Beschichtung:

| | |
|---|---|
| Hydroxypropylmethylcellulose (Cellulose-HP-M-603) | 3,3 mg |
| Glycerylpolyethylenglycoloxystearat (Cremophor RH 40) | 0,2 mg |
| Eisenoxid, red. | 0,2 mg |
| Talkum (PH) | 2,9 mg |
| Titandioxid (PH) | 0,4 mg |
| | 7 mg |
| Gesamtgewicht | 247 mg |

Formulierungsbeispiel 3:

Filmdragées enthaltend als Wirkstoff neutrales (+)-1-Isopropylamino-3-[o-(pyrrol-1-yl)-phenoxy]-2-propanol-fumarat oder racemisches 1-Isopropylamino-3-[o-(pyrrol-1-yl)-phenoxy]-2-propanol-hydrochlorid

Zusammensetzung:

Kern:

| | |
|---|---|
| Wirkstoff | 4,0 mg |
| Silica-Aerogel (Aerosil 200) | 2,5 mg |
| Cellulose (Avicel PH 102) | 36,0 mg |
| Laktose, krist. | 180,0 mg |
| Magnesiumstearat | 1,5 mg |
| Natriumcarboxymethylstärke | 16,0 mg |
| | 240,0 mg |

Beschichtung:

| | |
|---|---|
| Hydroxypropylmethylcellulose (Cellulose-HP-M-603) | 3,3 mg |
| Glycerylpolyethylenglycoloxystearat (Cremophor RH 40) | 0,2 mg |
| Eisenoxid, red. | 0,2 mg |
| Talkum (PH) | 2,9 mg |
| Titandioxid (PH) | 0,4 mg |
| | 7,0 mg |
| Gesamtgewicht | 247,0 mg |

Formulierungsbeispiel 4:

Filmdragées enthaltend als Wirkstoff neutrales (+)-1-Isopropylamino-3-[o-(pyrrol-1-yl)-phenoxy]-2-propanol-fumarat oder racemisches 1-Isopropylamino-3-[o-(pyrrol-1-yl)-phenoxy]-2-propanol-hydrochlorid

Zusammensetzung:

Kern:

| | |
|---|---|
| Wirkstoff | 10,0 mg |
| Silica-Aerogel (Aerosil 200) | 2,5 mg |
| Cellulose (Avicel PH 102) | 34,0 mg |
| Laktose, krist. | 174,0 mg |
| Magnesiumstearat | 1,5 mg |
| Natriumcarboxymethylstärke | 18,0 mg |
| | 240,0 mg |

Beschichtung:

| | |
|---|---|
| Hydroxypropylmethylcellulose (Cellulose-HP-M-603) | 3,3 mg |
| Glycerylpolyethylenglycoloxystearat (Cremophor RH 40) | 0,2 mg |
| Eisenoxid, red. | 0,2 mg |
| Talkum (PH) | 2,9 mg |
| Titandioxid (PH) | 0,4 mg |
| | 7,0 mg |
| Gesamtgewicht | 247,0 mg |

Herstellungsweise:

Die Bestandteile des Kerns werden vermischt, und das homogene Gemisch wird in Tabletten gepresst. Die Tabletten werden mit dem Lack beschichtet, der aus den beschriebenen Bestandteilen besteht, die wiederum z.B. in Wasser gelöse oder suspendiert wurden.

In analoger Weise wie in den Formulierungsbeispielen 1 - 4 beschrieben können Zusammensetzungen mit unterschiedlichem Wirkstoffgehalt hergestellt werden, wobei das (+)-Enantiomere bzw. Racemat auch in freier Form oder in Form eines anderen pharmazeutisch verwendbaren, nichttoxischen Salzes eingearbeitet werden kann.

**Patentansprüche**

1. (+)-1-Isopropylamino-3-[o-(pyrrol-1-yl)-phenoxy]-2-propanol oder ein Salz, insbesondere ein pharmazeutisch verwendbares, nicht-toxisches Salz davon.

2. (+)-1-Isopropylamino-3-[o-(pyrrol-1-yl)-phenoxy]-2-propanol oder ein Salz, insbesondere ein pharmazeutisch verwendbares, nichttoxisches Salz davon zur Anwendung in einem Verfahren zur therapeutischen Behandlung des menschlichen oder tierischen Körpers.

3. Verfahren zur Herstellung von (+)-1-Isopropylamino-3-[o-(pyrrol-1-yl)-phenoxy]-2-propanol und seiner Salze, dadurch gekennzeichnet, dass man

a) racemisches 1-Isopropylamino-3-[o-(pyrrol-1-yl)-phenoxy]-2-propanol oder ein Salz davon in das (+)-Enantiomere auftrennt, oder

b) ein Enantiomeres der Formel

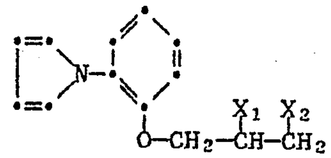 (I),

worin einerseits $X_1$ und $X_2$ gemeimsam für -O- stehen oder andererseits $X_1$ Hydroxy und $X_2$ reaktiosnfähiges verestertes Hydroxy, vor allem Halogen oder Sulfonyloxy, bedeutet, mit Isopropylamin umsetzt, oder

c) das (R)-Enantiomere der Formel

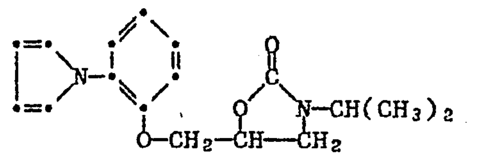 (II)

hydrolisiert, oder

d) von der (R)-enantiomeren Verbindung der Formel

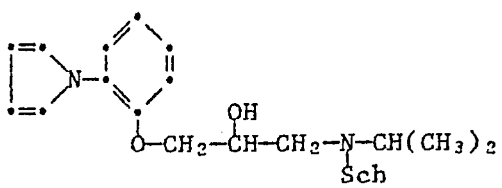 (III),

worin Sch eine üblicherweise zum Schutz von Aminogruppen verwendete Gruppe bedeutet, die Schutzgruppe abspaltet, oder

e) ausgehend von dem (R)-Enantiomeren der Formel

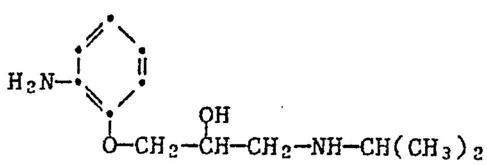 (IV),

den Pyrrol-1-yl-Ring aufbaut, und/oder, erhältliches freies (+)-1-Isopropylamino-3-[o-(pyrrol-1-yl)-phenoxy]-2-propanol in ein Salz überführt oder ein erhältliches Salz von (+)-1-Isopropylamino-3-[o-(pyrrol-1-yl)-phenoxy]-2-propanol in das freie Enantiomere überführt.

4. Pharmazeutische Präparate, enthaltend als Wirkstoff (+)-1-Isopropylamino-3-[o-(pyrrol-1-yl)-phenoxy]-2-propanol oder ein pharmazeutisch verwendbares, nichttoxisches Salz davon.

5. Pharmazeutische Präparate gemäss Anspruch 4 zur Behandlung von Angstzuständen, enthaltend als Wirkstoff (+)-1-Isopropylamino-3-[o-(pyrrol-1-yl)-phenoxy]-2-propanol oder ein pharmazeutisch verwendbares, nichttoxisches Salz davon.

6. Pharmazeutische Präparate gemäss einem der Ansprüche 4 oder 5, dadurch gekennzeichnet, dass als Wirkstoff das Fumarat von (+)-1-Isopropylamino-3-[o-(pyrrol-1-yl)-phenoxy]-2-propanol eingesetzt wird.

7. Pharmazeutische Präparate gemäss einem der Ansprüche 4 - 6, dadurch gekennzeichnet, dass der Wirkstoff in Tagesdosen von kleiner gleich 40 mg p.o., insbesondere von etwa 10 bis etwa 0,5 mg p.o., in erster Linie 3,5 bis 0,5 mg p.o., verabreicht wird.

8. Verwendung von (+)-1-Isopropylamino-3-[o-(pyrrol-1-yl)-phenoxy]-2-propanol oder eines pharmazeutisch verwendbaren, nichttoxischen Salzes davon zur Herstellung von pharmazeutischen Präparaten zur Behandlung von Angstzuständen.

9. Pharmazeutische Präparate zur Behandlung von Angstzuständen enthaltend als Wirkstoff 1-Isopropylamino-3-[o-(pyrrol-1-yl)-phenoxy]-2-propanol oder ein pharmazeutisch verwendbares, nichttoxisches Salz davon.

10. Pharmazeutische Präparate nach Anspruch 9, dadurch gekennzeichnet, dass das Hydrochlorid von 1-Isopropylamino-3-[o-(pyrrol-1-yl)-phenoxy]-2-propanol als Wirkstoff eingesetzt wird.

11. Pharmazeutische Präparate nach Anspruch 9 oder 10, dadurch gekennzeichnet, dass der Wirkstoff in Tagesdosen kleiner gleich 40 mg p.o., in erster Linie von 3,5 bis 0,5 mg p.o. verabreicht wird.

12. Verwendung von 1-Isopropylamino-3-[o-(pyrrol-1-yl)-phenoxy]-2-propanol oder eines pharmazeutisch verwendbaren, nichttoxischen Salzes davon zur Herstellung von pharmazeutischen Präparaten nach einem der Ansprüche 9 bis 11.

Patentansprüche für die folgenden Vertragsstaten: ES und GR.

1. Verfahren zur Herstellung von (+)-1-Isopropylamino-3-[o-(pyrrol-1-yl)-phenoxy]-2-propanol und seiner Salze, dadurch gekennzeichnet, dass man
a) racemisches 1-Isopropylamino-3-[o-(pyrrol-1-yl)-phenoxy]-2-propanol oder ein Salz davon in das (+)-Enantiomere auftrennt, oder
b) ein Enantiomeres der Formel

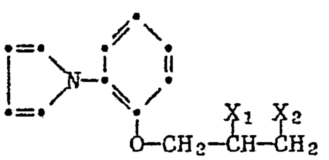

(I),

worin einerseits $X_1$ und $X_2$ gemeinsam für -O- stehen oder andererseits $X_1$ Hydroxy und $X_2$ reaktiosnfähiges verestertes Hydroxy, vor allem Halogen oder Sulfonyloxy, bedeutet, mit Isopropylamin umsetzt, oder
c) das (R)-Enantiomere der Formel

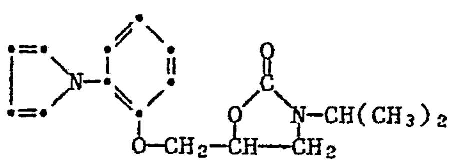

(II)

hydrolisiert, oder
d) von der (R)-enantiomeren Verbindung der Formel

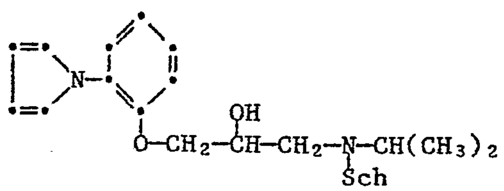

(III),

worin Sch eine üblicherweise zum Schutz von Aminogruppen verwendete Gruppe bedeutet, die Schutzgruppe abspaltet, oder
e) ausgehend von dem (R)-Enantiomeren der Formel

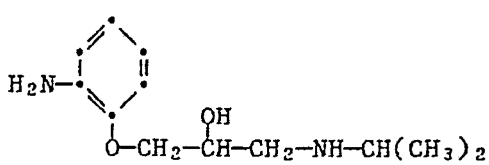

(IV),

den Pyrrol-1-yl-Ring aufbaut, und/oder, erhältliches freies (+)-1-Isopropylamino-3-[o-(pyrrol-1-yl)-phenoxy]-2-propanol in ein Salz überführt oder ein erhältliches Salz von (+)-1-Isopropylamino-3-[o-(pyrrol-1-yl)-phenoxy]-2-propanol in das freie Enantiomere überführt.

2. Verwendung von (+)-1-Isopropylamino-3-[o-(pyrrol-1-yl)-phenoxy]-2-propanol oder eines pharmazeutisch verwendbaren, nichttoxischen Salzes davon zur Herstellung von pharmazeutischen Präparaten zur Behandlung von Angstzuständen.

3. Verfahren zur Herstellung von pharmazeutischen Präparaten, dadurch gekennzeichnet, dass man (+)-1-Isopropylamino-3-[o-(pyrrol-1-yl)-phenoxy]-2-propanol oder ein pharmazeutisch verwendbares, nichttoxisches Salz davon gegebenenfalls unter Beimischung von üblichen Hilfs- und Trägerstoffen zu entsprechenden Präparaten verarbeitet.

4. Verfahren gemäss Anspruch 3 zur Herstellung von pharmazeutischen Präparaten zur Behandlung von Angstzuständen.

5. Verfahren gemäss Anspruch 3 oder 4, dadurch gekennzeichnet, dass als pharmazeutisch verwendbares Salz das Fumarat von (+)-1-Isopropylamino-3-[o-(pyrrol-1-yl)-phenoxy]-2-propanol eingesetzt wird.

6. Verfahren gemäss Anspruch 3, 4 oder 5, dadurch gekennzeichnet, dass der Wirkstoff in Tagesdosen von kleiner gleich 40 mg p.o., insbesondere von etwa 10 bis etwa 0,5 mg p.o., in erster Linie 3,5 bis 0,5 mg p.o., eingearbeitet wird.

7. Verfahren gemäss Anspruch 6, dadurch gekennzeichnet, dass der Wirkstoff in einer Tagesdosis von 2 mg p.o. eingearbeitet wird.

8. Verfahren zur Herstellung von pharmazeutischen Präparaten zur Behandlung von Angstzuständen, dadurch gekennzeichnet, dass man 1-Isopropylamino-3-[o-(pyrrol-1-yl)-phenoxy]-2-propanol oder ein pharmazeutisch verwendbares, nicht-toxisches Salz davon gegebenenfalls unter Beimischung von üblichen Hilfs- und Trägerstoffen zu entsprechenden Präparaten verarbeitet.

9. Verfahren nach Anspruch 8, dadurch gekennzeichnet, dass das Hydrochlorid von 1-Isopropylamino-3-[o-(pyrrol-1-yl)-phenoxy]-2-propanol als Wirkstoff eingesetzt wird.

10. Verfahren nach Anspruch 8 oder 9, dadurch gekennzeichnet, dass der Wirkstoff in Tagesdosen kleiner gleich 40 mg p.o., in erster Linie von 3,5 bis 0,5 mg p.o. eingearbeitet wird.

11. Verfahren nach einem der Ansprüche 8 - 10, dadurch gekennzeichnet, dass der Wirkstoff in Tagesdosen von 2 mg p.o. eingearbeitet wird.

12. Verwendung von 1-Isopropylamino-3-[o-(pyrrol-1-yl)-phenoxy]-2-propanol oder eines pharmazeutisch verwendbaren, nichttoxischen Salzes davon zur Herstellung von pharmazeutischen Präparaten nach einem der Ansprüche 8 bis 11.

Europäisches Patentamt

# EUROPÄISCHER RECHERCHENBERICHT

Nummer der Anmeldung

EP 87 81 0604

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | DE-A-2 409 313 (CIBA-GEIGY AG)<br>* Insgesamt *<br>----- | 1-12 | C 07 D 207/325<br>A 61 K 31/40 |
| | | | **RECHERCHIERTE SACHGEBIETE (Int. Cl.4)** |
| | | | C 07 D 207/00 |

Der vorliegende Recherchenbericht wurde für alle Patentansprüche erstellt

| Recherchenort | Abschlußdatum der Recherche | Prüfer |
|---|---|---|
| DEN HAAG | 20-01-1988 | MAISONNEUVE J.A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)